# EUROPEAN PATENT APPLICATION

(11) **EP 4 570 244 A1**
(43) Date of publication of application: **18.06.2025**
(21) Application number: 23852304.7
(22) Date of filing: 13.07.2023
(51) Int. Cl.: A61K 31/085, A23L 33/10, A61K 35/618, A61P 13/12, A61P 39/06

(54) **ENHANCING AGENT OF CARDIOLIPIN MOLECULAR SPECIES THAT IS MITOCHONDRIAL COMPONENT**

(30) Priority: 09.08.2022 JP 2022126781
(71) Applicant: Watanabe Oyster Laboratory Co., Ltd., Hachioji-Shi Tokyo 192-0154 (JP)
(72) Inventor: WATANABE Mitsugu, Hachioji-shi, Tokyo 192-0154 (JP); CHIBA Hitoshi, Sapporo-shi, Hokkaido 060-0808 (JP); KEI Shukuhei, Sapporo-shi, Hokkaido 060-0808 (JP); KA Kin-you, Sapporo-shi, Hokkaido 060-0808 (JP); SAKURAI Toshihiro, Sapporo-shi, Hokkaido 060-0808 (JP); WATANABE Hideaki, Hachioji-shi, Tokyo 192-0154 (JP); WATANABE Takayuki, Hachioji-shi, Tokyo 192-0154 (JP)
(74) Representative: Plougmann Vingtoft a/s
(86) International application number: PCT/JP2023/025917
(87) International publication number: WO 2024/034322

(57) **Abstract**

[Problem]

It is an objective of the present invention to provide an enhancer with useful actions such as increase action of cardiolipin molecular species as a component of an inner mitochondrial membrane including 3,5-dihydroxy-4-methoxybenzyl alcohol as an active ingredient.

[Solution]

The present invention features the increase action of cardiolipin molecular species as a component of an inner mitochondrial membrane including 3,5-dihydroxy-4-methoxybenzyl alcohol as an active ingredient.

## Description

### TECHNICAL FIELD

The present invention relates to various enhancers, such as an enhancer for cardiolipin molecular species, which are components of inner mitochondrial membranes under oxidative stress stimulation in human renal proximal tubule cells (HK-2).

### BACKGROUND ART

An oxidative stress state refers to a state in which a balance between oxidation and antioxidant functions within cells is disrupted, and excessive reactive oxygen species accumulate within cells. The oxidative stress state is a cause of various diseases, including aging and kidney disease.

On the other hand, when adenosine triphosphate, which is an energy source, is produced in mitochondria, the reactive oxygen species are produced as by-products. In a state of elevated oxidative stress, changes in the composition and decrease in the total amount of cardiolipin, which is a phospholipid located in the mitochondrial inner membrane, have been reported, leading to functional impairment of intracellular proteins and cell death.

By the way, there are many mitochondria in the proximal tubule cells of the kidneys. The reason for this is thought to be that a large amount of the adenosine triphosphate produced by the mitochondria is required when reabsorbing nutrients and other substances.

Therefore, it is thought that the cardiolipin composition of the mitochondria plays an important role in the proximal tubule cells of the kidneys. This is because, as described above, in the state of elevated oxidative stress, changes in the composition and the decrease in the total amount of the cardiolipin, which is a phospholipid located in the mitochondrial inner membrane, have been reported.

Japanese oyster (Magaki), which is a bivalve belonging to *Pterioida Ostreidae,* and its habitat extends to the whole region of East Asia, including Japan. In addition to glycogen and protein, Magaki is a highly nutritious food that contains large amounts of minerals such as calcium and zinc. The present inventors of this case explored and researched for biologically active substances in Magaki, and as a result of exploring for substances with antioxidant activity in Magaki extract, they discovered the antioxidant substance, which is 3,5-dihydroxy-4-methoxybenzyl alcohol (hereinafter referred to as DHMBA). To date, the radical scavenging ability of DHMBA and the activation of the Keap1-Nrf2 pathway and the induction of the expression of antioxidant gene groups (such as HO-1 and NQO1) that accompany this have been confirmed.

The former is called direct antioxidant activity, and the latter is called indirect antioxidant activity, and it has been demonstrated that DHMBA is a substance that has both functions. Furthermore, it has been found that DHMBA has lower cytotoxicity than existing antioxidants. Therefore, the usefulness of DHMBA in kidney protection is further verified.

Patent Document 1: JP-A-2017-132753

### DISCLOSURE OF THE INVENTION

### PROBLEMS TO BE SOLVED BY THE INVENTION

The present invention is a further development of the invention of each useful agent that has 3,5-dihydroxy-4-methoxybenzyl alcohol as an active ingredient, which has already been obtained by the present inventors, and aims to provide various enhancers, such as an enhancer for cardiolipin molecular species, which are components of the inner mitochondrial membranes and have useful actions such as increase action of the cardiolipin molecular species as the components of the inner mitochondrial membrane, using 3,5-dihydroxy-4-methoxybenzyl alcohol as an active ingredient.

### SOLUTIONS TO THE PROBLEMS

The present invention includes 3,5-dihydroxy-4-methoxybenzyl alcohol as an active ingredient and has an increase action on cardiolipin molecular species as a component of an inner mitochondrial membrane.

Alternatively, the present invention includes 3,5-dihydroxy-4-methoxybenzyl alcohol as an active ingredient and has an increase action on cardiolipin molecular species as a component of an inner mitochondrial membrane under oxidative stress stimulation in human renal proximal tubule cells (HK-2).

Alternatively, the present invention includes 3,5-dihydroxy-4-methoxybenzyl alcohol as an active ingredient and has an increase action on total cardiolipin content as a component of an inner mitochondrial membrane.

Alternatively, the present invention includes 3,5-dihydroxy-4-methoxybenzyl alcohol as an active ingredient and has an increase action on total cardiolipin content as a component of an inner mitochondrial membrane under oxidative stress stimulation in human renal proximal tubule cells (HK-2).

Alternatively, the present invention includes 3,5-dihydroxy-4-methoxybenzyl alcohol as an active ingredient and has an increase action on unsaturated fatty acid chains through acyl group modification of cardiolipin as a component of an inner mitochondrial membrane.

Alternatively, the present invention includes 3,5-dihydroxy-4-methoxybenzyl alcohol as an active ingredient and has an increase action on unsaturated fatty acid chains through acyl group modification of cardiolipin as a component of an inner mitochondrial membrane under oxidative stress stimulation in human renal proximal tubule cells (HK-2).

Alternatively, the present invention includes 3,5-dihydroxy-4-methoxybenzyl alcohol as an active ingredient and has an activity on biosynthesis, remodeling, and degradation of cardiolipin as a component of an inner mitochondrial membrane.

Alternatively, the present invention includes 3,5-dihydroxy-4-methoxybenzyl alcohol as an active ingredient and has an activity on biosynthesis, remodeling, and degradation of cardiolipin as a component of an inner mitochondrial membrane under oxidative stress stimulation in human renal proximal tubule cells (HK-2).

### EFFECTS OF THE INVENTION

The present invention has an excellent effect of being able to provide an enhancer that has useful actions such as the increase action of cardiolipin molecular species, which are components of inner mitochondrial membranes, using 3,5-dihydroxy-4-methoxybenzyl alcohol as an active ingredient.

### BRIEF DESCRIPTION OF THE DRAWINGS

FIG. 1 is an explanatory drawing for describing a strategy for exploring a cardiolipin (CL) increase effect.
FIG. 2 is an explanatory drawing for describing increase effects of DHMBA on CL molecular species under oxidative stress conditions. HK-2 cells were cultured with 100 µM BSO and 250 or 500 µM DHMBA, and then recovered and CL was measured using LC-MS/MS. Mean ± SD (n = 4).
FIG. 3 is an explanatory drawing for describing increase effects of DHMBA on total CL content under oxidative stress conditions. HK-2 cells were cultured with 100 µM BSO and 250 or 500 µM DHMBA, and then recovered and CL was measured using LC-MS/MS. Mean ± SD (n = 4).
FIG. 4 is an explanatory drawing for describing CL acyl group modification due to addition of DHMBA under oxidative stress conditions. HK-2 cells were cultured with 100 µM BSO and 250 or 500 µM DHMBA, and then recovered and CL was measured using LC-MS/MS. Mean ± SD (n = 4).
FIG. 5 is an explanatory drawing for describing changes in expression level of CL biosynthesis-related genes of DHMBA under oxidative stress conditions. HK-2 cells were cultured with 100 µM BSO and 250 or 500 µM DHMBA, and then the expression levels of CL biosynthesis-related genes were measured by qPCR. Mean ± SD (n = 4).
FIG. 6 is an explanatory drawing for describing changes in the expression levels of genes related to CL remodeling and degradation of DHMBA under oxidative stress conditions. HK-2 cells were cultured with 100 µM BSO and 250 or 500 µM DHMBA, and then the expression levels of genes related to mitochondria biosynthesis were measured by qPCR. Mean ± SD (n = 4).
FIG. 7 is an explanatory drawing for describing with Table 1 describing primer sequences used in the present invention.

### DESCRIPTION OF PREFERRED EMBODIMENTS

### (EXAMPLES)

The following is a description of the present invention based on one example illustrated in the figures.

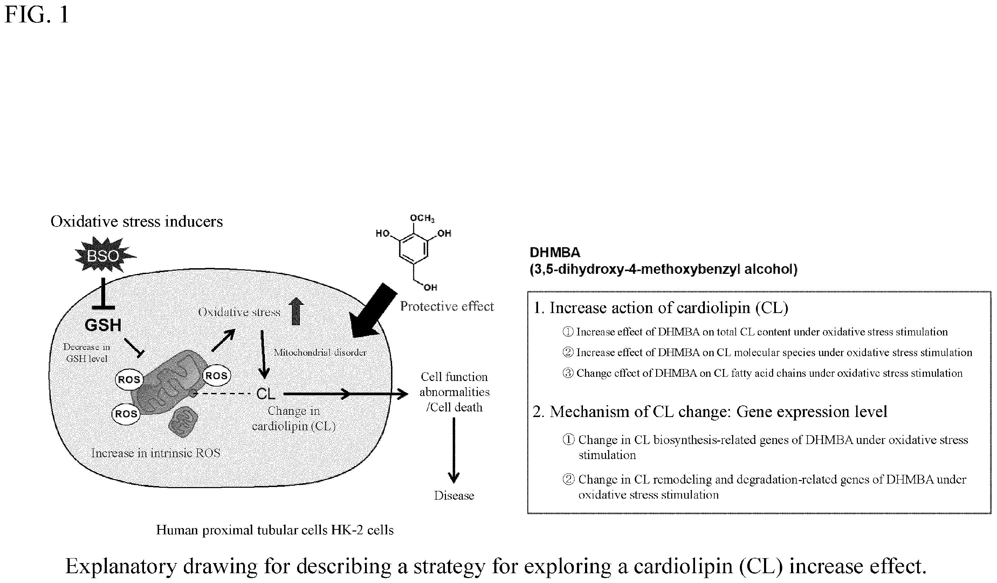

In the present invention, it was focused on mitochondria, and a strategy for exploring a cardiolipin increase effect of DHMBA using human renal proximal tubule cells HK-2 (FIG. 1) was verified.

As illustrated in FIG. 1, when oxidative stress occurs in the human renal proximal tubule cells HK-2 using, for example, an oxidative stress inducer, glutathione content decreases.

Here, the oxidative stress occurs when there is an excess of free radicals or reactive oxygen species in the body. It has been found that the accumulation of the excessive reactive oxygen species leads to cellular disorder, such as damage to DNA, proteins, and lipid membranes. The damage to cells caused by the reactive oxygen species has been suggested to be related to onset of many pathological conditions, including aging, asthma, arthritis, diabetes, cancer, inflammation, cardiovascular disease, atherosclerosis, Down syndrome, and neurodegenerative disease.

Glutathione is an important tripeptide thiol in cells, and is composed of glutamic acid, cysteine, and glycine.

Glutathione also acts as an antioxidant, protecting the cells from damage caused by free radicals. Intracellular glutathione exists in forms of reduced glutathione (GSH) and oxidized glutathione (GSSG). In normal cells and tissues, the reduced glutathione (GSH) accounts for 90% of total glutathione, and the oxidized glutathione (GSSG), which is a disulfide type, accounts for equal to or less than 10%.

The reason for the high proportion of reduced glutathione (GSH) is that enzymes that convert the oxidized glutathione (GSSG), which are glutathione reductase (GSH reductase), are always active and induced by the oxidative stress, and the rise in the GSSG/GSH ratio is also thought of as an indicator of the oxidative stress.

As illustrated in FIG. 1, when the glutathione content decreases, intrinsic reactive oxygen species increase, leading to disorder to mitochondria. This also causes change in the cardiolipin content, leading to abnormal cell function or cell death.

Therefore, the present inventors of this case focused on mitochondria and used the human renal proximal tubule cells HK-2 to verify the cardiolipin increase effect of DHMBA, that is, the increase effect on total cardiolipin content when DHMBA is added under oxidative stress stimulation, the increase effect on cardiolipin molecular weight when DHMBA is added under oxidative stress stimulation, and the change effect on cardiolipin fatty acid chains when DHMBA is added under oxidative stress stimulation. Furthermore, a mechanism of the cardiolipin change: gene expression levels, that is, the change in the expression levels of genes related to the cardiolipin biosynthesis when DHMBA is added under oxidative stress stimulation, and the change in the expression levels of genes related to cardiolipin remodeling and degradation when DHMBA is added under oxidative stress stimulation, were verified.

"Verification experiments to explore the cardiolipin increase effect and the like"

### (Materials and Methods)

### Cell culture

Human renal proximal tubule cells (HK-2) were subcultured in Dulbecco's Modified Eagle's Medium (DMEM, Nacalai Tesque) containing 10% fetal bovine serum and 1% penicillin-streptmycin at 37°C in a 5% CO₂ incubator. The following experiments were conducted using the oxidative stress inducer to confirm the cardiolipin increase effect of DHMBA on the oxidative stress.

### "Verification of total cardiolipin content in cells"

HK-2 cells were seeded in a 6-well plate at a density of 1.0 × 10⁵ cells/well and cultured for 24 hours. The cells were then divided into four groups with different stimulus conditions, and 2 mL thereof was added to each well.

The above-described four groups with the different stimulus conditions are as follows.

Control group (culture medium); oxidative stress group (final concentration 100 µM Oxidative Stress); oxidative stress + DHMBA 250 group (final concentration 100 µM Oxidative Stress + final concentration 250 µM DHMBA); and oxidative stress + DHMBA 500 group (final concentration 100 µM Oxidative Stress + final concentration 500 µM DHMBA).

After 24 hours, the cells were washed twice with phosphate-buffered saline (hereinafter referred to as PBS), and then 1 mL of PBS was added to each well and the cells were recovered using a scraper. In this cell suspension, 100 µL was used for protein quantification by a bicinchoninic acid (BCA) method. The protein concentration was used for correction of the cardiolipin concentration. The remaining cell suspension was centrifuged at 1,200 rpm for 5 minutes, the supernatant was removed, and then the precipitated cells were stored at -80°C.

For the cardiolipin measurement, the total intracellular lipids were extracted in the same way as in the previous reports (Chen Z, et al. Metabolomics, 16: 115, 2020).

In short, the lipids in the sample were extracted using hexane/isopropanol 3:2 (v/v, with 0.05% butylated hydroxytoluene), and then dried and hardened in an evaporator. Next, the sample was dissolved in methanol (MeOH), and insoluble substances were removed by centrifugation at 15,000 rpm for 15 minutes at 4°C. The total lipids extracted were stored at -80°C. The cardiolipin measurement was performed using LC-MS/MS, following the same procedure as before.

"Expression levels of genes related to cardiolipin biosynthesis, remodeling and degradation"

For example, it is reported that the oxidative stress stimulation using the oxidative stress inducer induces the changes in the composition of the cardiolipin molecular species and the decrease in the total cardiolipin content, and at the same time induces functional impairment of the mitochondrial membrane proteins, including those of the electron transport chain, and apoptosis, which is one form of cell death (Paradies G, et al. FEBS Lett, 466: 323-326, 2000; Paradies G, et al. Cells, 8: 728, 2019).

Therefore, in the present invention, the analysis of the expression levels of genes related to cardiolipin biosynthesis, remodeling, and degradation in response to oxidative stress stimulation was verified.

HK-2 cells were seeded in a 6-well plate at a density of 5.0 × 10⁴ cells/well and cultured for 24 hours.

Next, under the same stimulus conditions as described above, 2 mL thereof was added to each well. After 24 hours, total RNA was recovered using NucleoSpin (registered trademark) RNA Kit (MACHEREY-NAGEL). The concentration of the purified RNA was quantified by Nanodrop (Invitrogen). From 1.0 µg of the total RNA, cDNA was synthesized according to a protocol for ReverTra Ace (registered trademark) qPCR RT Master Mix with gDNA Remover (TOYOBO).

The gene expression levels were measured according to a protocol for THUNDERBIRD (registered trademark) SYBR (registered trademark) qPCR Mix (TOYOBO). Measurements were performed using a CFX Connect^{™} real-time PCR analysis system (BioRad), and analyzed using a 2^{-(ΔΔCT)} method.

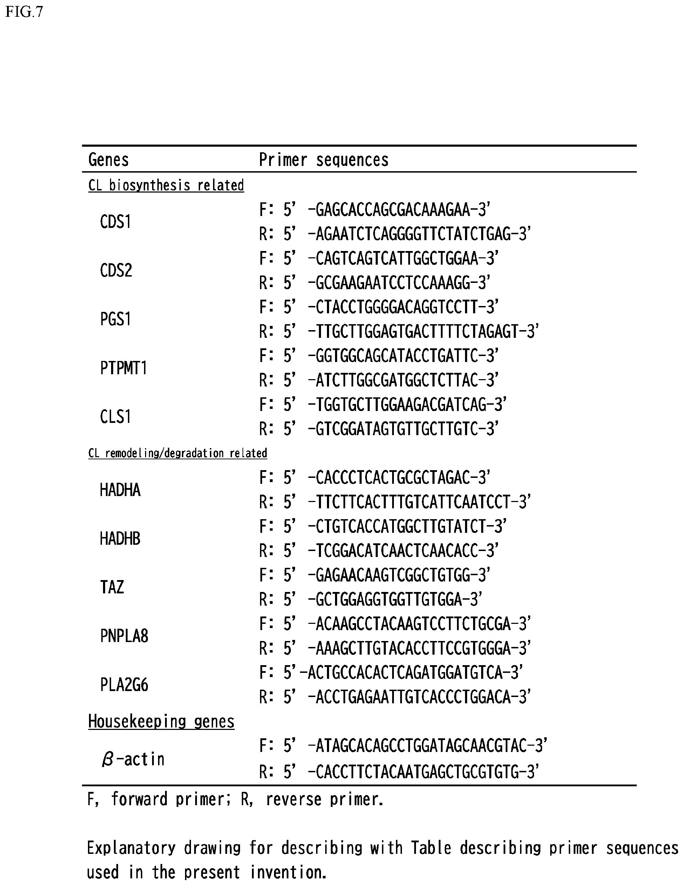

The gene expression level of the control group was set at 1 for comparison. The figures are expressed as mean ± SD (n = 4 for each group). Each expression level was corrected using the expression level of a housekeeping gene β-actin. The sequences of primers used are shown in a table in FIG. 7.

### "Statistical processing"

The data obtained were expressed as mean ± standard deviation (SD), and statistical analysis was performed using JMP Pro 16 (SAS Institute Inc.). The Dunnett test was used for multiple comparisons between the groups, and P < 0.05 was set as the statistical significance level.

### "Results and discussion"

### (Changes in Cardiolipin Molecular Species)

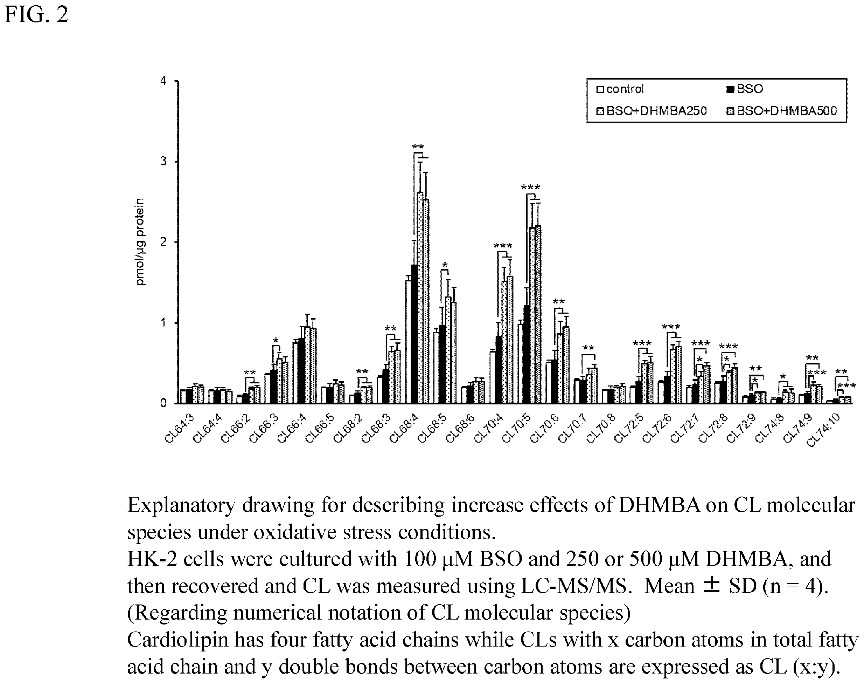

The results so far have shown that DHMBA has a protective effect on mitochondria under oxidative stress conditions. Here, the changes in cardiolipin as an important component of the inner mitochondrial membranes (FIG. 2) were investigated.

In the oxidative stress additive group, there was no significant fluctuation in each of the cardiolipin molecular species compared to the control group, but in the oxidative stress + DHMBA additive group, almost all the cardiolipin molecular species increased significantly. The cardiolipin molecular species correlate with mitochondrial function and cell function, and from these results, it was thought that the mitochondrial function rose due to the addition of DHMBA.

### (Change in Total Cardiolipin Content)

Since almost all the cardiolipin molecular species increased when DHMBA was added, the changes in the total cardiolipin content were also investigated.

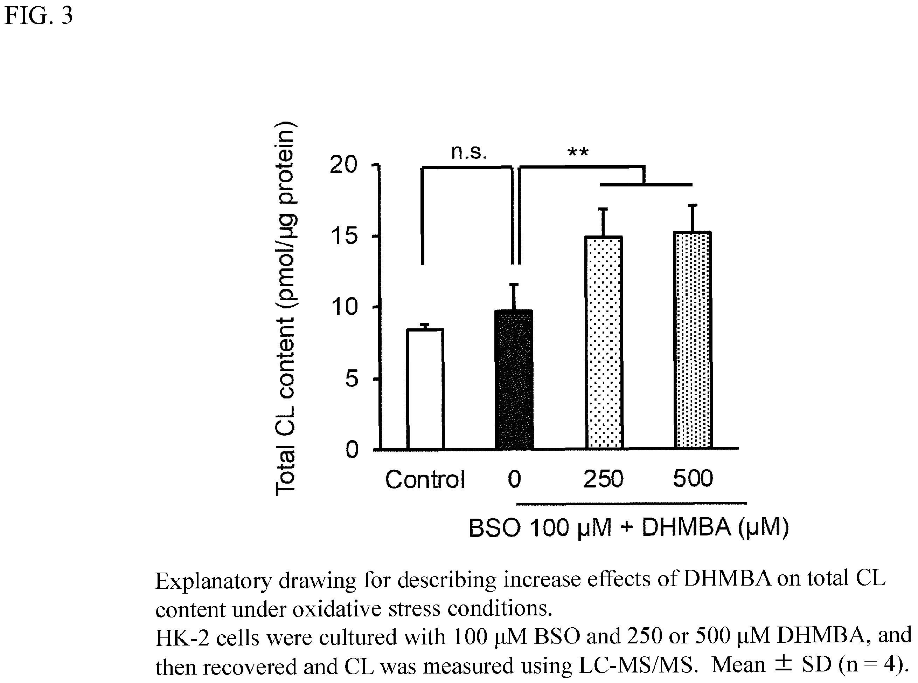

As illustrated in FIG. 3, no decrease in the total cardiolipin content was observed in HK-2 cells treated with 100 µM Oxidative Stress (100 µM Oxidative Stress + 0 µM DHMBA group).

On the other hand, co-treatment with 100 µM oxidative stress and 250 µM or 500 µM DHMBA increased the total cardiolipin content by about 1.5 times.

The results suggest that the oxidative stress stimulation did not cause any change in the total cardiolipin content. However, DHMBA increased the total cardiolipin content. These results are consistent with the changes in the cardiolipin molecular species shown in FIG. 2. Although the oxidative stress stimulation did not cause any change in the cardiolipin molecular species or the total cardiolipin content, DHMBA increased both the cardiolipin molecular species and the total cardiolipin content, suggesting that the mitochondrial function was improved by the addition of DHMBA.

### (Cardiolipin Acyl Group Modification)

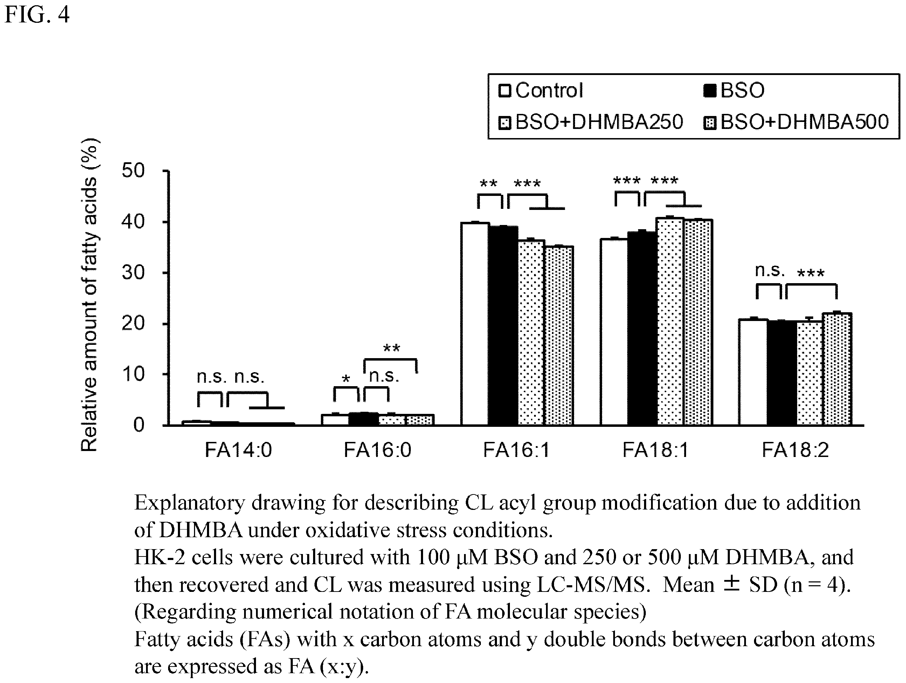

Furthermore, it has been reported that the compositions of the acyl groups of cardiolipin are related to the mitochondrial function (Chicco AJ and Sparagna GC. Am J Physiol Cell Physiol, 292: C33-C44, 2007), and in the verification of the present invention, the distribution of the acyl groups of cardiolipin were also investigated (FIG. 4).

The addition of the oxidative stress increased the saturated fatty acid chain FA16:0, and the addition of DHMBA decreased the saturated fatty acid chain FA16:0 and increased the unsaturated fatty acid chains FA18:1 and FA18:2.

The increase in the unsaturated fatty acid chains is thought to have improved the fluidity of the inner mitochondrial membranes and raised the function of the membrane proteins (Fouret G, et al. Biochim Biophys Acta Bioenerg, 1847: 1025-1035, 2015).

Therefore, it is thought that the change in the cardiolipin profile caused by DHMBA can explain the protective effect of DHMBA on the mitochondria.

As for the results of the CL acyl group modification, the fatty acids are classified into two types, "saturated fatty acids" and "unsaturated fatty acids," depending on their difference in structure. The fatty acids with no double bonds between the carbons are called saturated fatty acids, and the fatty acids with the double bonds are called unsaturated fatty acids.

In the results, it says, "The addition of the oxidative stress increased the saturated fatty acid chains FA16:1 and FA16:0, and the addition of DHMBA decreased the saturated fatty acid chain FA16:0 and increased the unsaturated fatty acid chains FA18:1 and FA18:2." It is known that the more the phospholipids with a large number of double bonds (which means a high degree of unsaturation) are contained, the higher the fluidity of the lipid bilayer. The reason for this is that, since they are unsaturated fatty acids, the fatty acids bend, creating gaps between adjacent phospholipids, and it is thought that this makes a soft membrane having fluidity.

Therefore, "the increase in the unsaturated fatty acid chains is thought to have improved the fluidity of the inner mitochondrial membranes and raised the function of the membrane proteins (Fouret G, et al. Biochim Biophys Acta Bioenerg, 1847: 1025-1035, 2015)" thanks to the addition of DHMBA.

### (Gene Expression Level related to Biosynthesis, Remodeling, and Degradation of Cardiolipin)

In order to investigate the mechanism of the DHMBA-induced increases in cardiolipin, the expression of genes related to cardiolipin biosynthesis, remodeling, and degradation was verified.

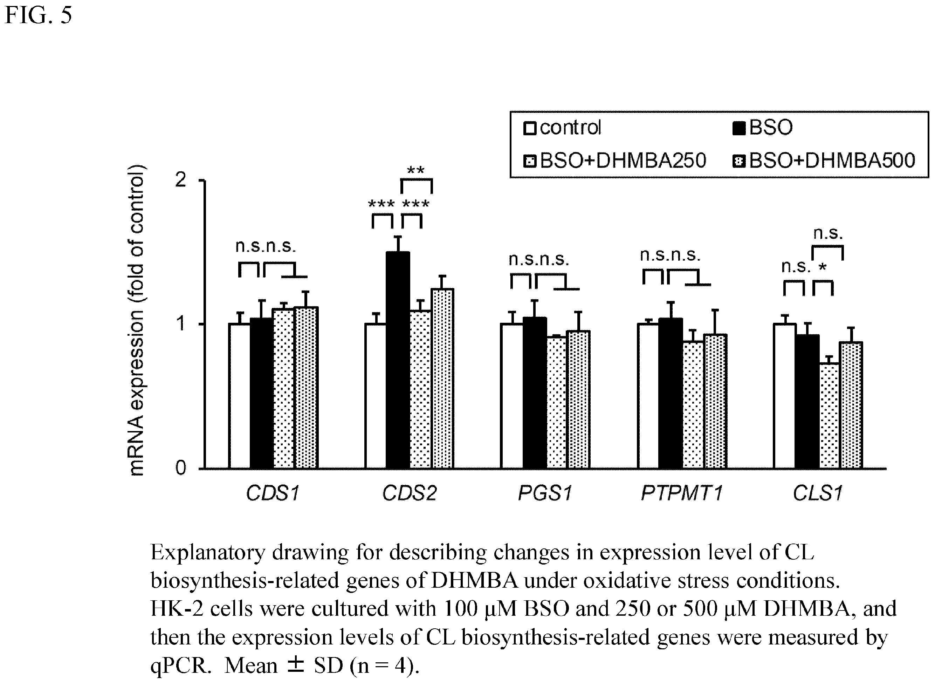

First, as shown in FIG. 5, in the oxidative stress group, the gene expression level of CDS2, which is related to the cardiolipin biosynthesis, increased. However, it decreased to the same level as the control group when DHMBA was added.

Here, CDS2 is one of the synthetic enzymes that work in the CL biosynthesis pathway, and CDP-DAG is synthesized from phosphatidic acid (PA) and cytidine triphosphate (CTP) by CDS. The activity is known to exist in the endoplasmic reticulum membrane and inner mitochondrial membranes. Two genes, CDS1 and CDS2, have been identified as CDS.

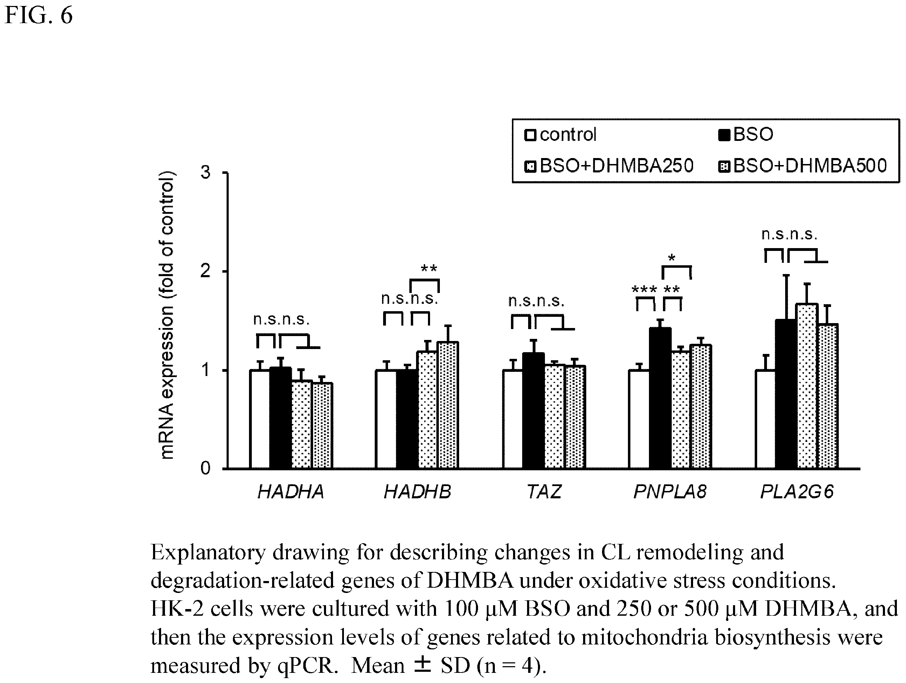

In addition, the gene expression level of PNPLA8, which is related to the cardiolipin remodeling and the degradation, also increased in the oxidative stress group, and decreased to the same level as the control group when DHMBA was added (FIG. 6).

Here, PNPLA8 refers to the enzyme that cleaves the 2nd position of phospholipase A (PLA), which cleaves fatty acids. This enzyme is localized in mitochondria and peroxisomes, and is involved in the metabolism of the oxidized cardiolipin, and is important for maintaining the mitochondrial function. Therefore, defects or mutations in PNPLA8 are related to lipid metabolism abnormalities and mitochondrial disorders, and cause phenotypes such as muscle weakness, fat tissue atrophy, a decrease in body temperature, and neurodegeneration, mainly in skeletal muscle, myocardium, adipose tissue, and cerebral nervous system, which are areas with high energy metabolism.

It has been shown that the cardiolipin biosynthesis, the remodeling, and the degradation are activated under oxidative stress conditions, and it is thought that HK-2 cells that have suffered the oxidative stress disorder may have attempted to maintain the mitochondrial function through the synthesis of the mitochondria and the hyperactive remodeling. Therefore, it is possible that the addition of the oxidative stress did not cause any change in the total cardiolipin content or its composition.

On the other hand, the addition of DHMBA caused a decrease in CDS2, which is related to the cardiolipin biosynthesis, and the expression levels of PNPLA8 and HADHB, which are related to the cardiolipin remodeling and the degradation, respectively, decreased and increased. Although the relationship between the changes in the gene expression levels related to the cardiolipin biosynthesis, the remodeling, and the degradation and the changes in the cardiolipin profile is not clear, it can be interpreted that DHMBA decreases the oxidative stress, eliminating the need for the compensatory action of the oxidative stress (change in gene expression levels) in HK-2 cells.

Here, a description of 3-hydroxy acyl-CoA dehydrogenase (HADH) is given. Long-chain fatty acids taken up into the cells are metabolized sequentially by the respective dehydrogenases in the mitochondria according to the carbon length of the fatty acids, and at each step, the carbon chain is shortened by two units to reach acetyl-CoA, which contributes to energy production.

3-hydroxy acyl-CoA dehydrogenase (HADH) is an enzyme that exists in the inner mitochondrial membranes and is responsible for the metabolism of medium- and short-chain acyl-CoA in cooperation with other β-oxidation enzyme group.

The results suggest that the addition of DHMBA held and improved the mitochondrial function by many cardiolipins maintaining the functionality.

### (Regarding the Manufacturing Method of the Present Invention)

Manufacturing methods for an enhancer for cardiolipin molecular species as a component of an inner mitochondrial membrane, an enhancer for total cardiolipin content as a component of an inner mitochondrial membrane, an enhancer for total cardiolipin content as a component of an inner mitochondrial membrane, an enhancer for unsaturated fatty acid chains through acyl group modification of cardiolipin as a component of an inner mitochondrial membrane, an activator of biosynthesis, remodeling, and degradation of cardiolipin as a component of an inner mitochondrial membrane, and the like including the above-described 3,5-dihydroxy-4-methoxybenzyl alcohol as an active ingredient are described.

As described above, 3,5-dihydroxy-4-methoxybenzyl alcohol can be obtained from Magaki, and the present inventors of this case have already obtained many patents for manufacturing methods that efficiently extract 3,5-dihydroxy-4-methoxybenzyl alcohol from the above-described Magaki. 3,5-dihydroxy-4-methoxybenzyl alcohol can also be produced synthetically, and the present inventors have already obtained a patent for this synthetic method.

In the present invention, it was surmised that 3,5-dihydroxy-4-methoxybenzyl alcohol would be useful for protecting each organ in the human body, and experiments had been conducted to verify it, leading to the present invention.

Therefore, the useful agents of the present invention are manufactured after conducting these verifications.

## Claims

1. An enhancer for cardiolipin molecular species as a component of an inner mitochondrial membrane comprising
3,5-dihydroxy-4-methoxybenzyl alcohol as an active ingredient, the enhancer having an increase action on the cardiolipin molecular species as the component of the inner mitochondrial membrane.

2. An enhancer for cardiolipin molecular species as a component of an inner mitochondrial membrane comprising
3,5-dihydroxy-4-methoxybenzyl alcohol as an active ingredient, the enhancer having an increase action on the cardiolipin molecular species as the component of the inner mitochondrial membrane under oxidative stress stimulation in human renal proximal tubule cells (HK-2).

3. An enhancer for total cardiolipin content as a component of an inner mitochondrial membrane comprising
3,5-dihydroxy-4-methoxybenzyl alcohol as an active ingredient, the enhancer having an increase action on the total cardiolipin content as the component of the inner mitochondrial membrane.

4. An enhancer for total cardiolipin content as a component of an inner mitochondrial membrane comprising
3,5-dihydroxy-4-methoxybenzyl alcohol as an active ingredient, the enhancer having an increase action on the total cardiolipin content as the component of the inner mitochondrial membrane under oxidative stress stimulation in human renal proximal tubule cells (HK-2).

5. An enhancer for unsaturated fatty acid chains through acyl group modification of cardiolipin as a component of an inner mitochondrial membrane comprising
3,5-dihydroxy-4-methoxybenzyl alcohol as an active ingredient, the enhancer having an increase action on the unsaturated fatty acid chains through acyl group modification of cardiolipin as the component of the inner mitochondrial membrane.

6. An enhancer for unsaturated fatty acid chains through acyl group modification of cardiolipin as a component of an inner mitochondrial membrane comprising
3,5-dihydroxy-4-methoxybenzyl alcohol as an active ingredient, the enhancer having an increase action on the unsaturated fatty acid chains through acyl group modification of cardiolipin as the component of the inner mitochondrial membrane under oxidative stress stimulation in human renal proximal tubule cells (HK-2).

7. An activator of biosynthesis, remodeling, and degradation of cardiolipin as a component of an inner mitochondrial membrane comprising
3,5-dihydroxy-4-methoxybenzyl alcohol as an active ingredient, the activator having an activity on biosynthesis, remodeling, and degradation of cardiolipin as the component of the inner mitochondrial membrane.

8. An activator of biosynthesis, remodeling, and degradation of cardiolipin as a component of an inner mitochondrial membrane comprising
3,5-dihydroxy-4-methoxybenzyl alcohol as an active ingredient, the activator having an activity on biosynthesis, remodeling, and degradation of cardiolipin as the component of the inner mitochondrial membrane under oxidative stress stimulation in human renal proximal tubule cells (HK-2).
